(19) Europäisches Patentamt
European Patent Office
Office européen des brevets

(11) Veröffentlichungsnummer: **0 345 657**
**A2**

(12) # EUROPÄISCHE PATENTANMELDUNG

(21) Anmeldenummer: **89110003.4**

(51) Int. Cl.4: **C07D 265/30 , A01N 43/84 , //C07C43/12**

(22) Anmeldetag: **02.06.89**

(30) Priorität: **08.06.88 DE 3819465**

(43) Veröffentlichungstag der Anmeldung:
**13.12.89 Patentblatt 89/50**

(84) Benannte Vertragsstaaten:
**AT BE CH DE ES FR GB GR IT LI NL SE**

(71) Anmelder: **BASF Aktiengesellschaft**
**Carl-Bosch-Strasse 38**
**D-6700 Ludwigshafen(DE)**

(72) Erfinder: **Rentzea, Costin, Dr.**
**Richard-Kuhn-Strasse 1-3**
**D-6900 Heidelberg(DE)**
Erfinder: **Ammermann, Eberhard, Dr.**
**Sachsenstrasse 3**
**D-6700 Ludwigshafen(DE)**
Erfinder: **Lorenz, Gisela, Dr.**
**Erlenweg 13**
**D-6730 Neustadt(DE)**

(54) **Cyclische Amine und diese enthaltende Fungizide.**

**Amine der allgemeinen Formel I**

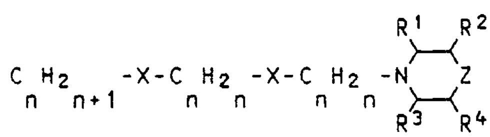

I

**worin**
**n die ganzen Zahlen 2 bis 10,**
**X Sauerstoff oder Schwefel,**
**Z Sauerstoff oder CH-$R_5$ bedeutet und**
**$R^1$, $R^2$, $R^3$, $R^4$ und $R^5$ Wasserstoff oder Alkyl bedeuten, sowie deren Salze und diese Verbindungen enthaltende Fungizide.**

**EP 0 345 657 A2**

## Cyclische Amine und diese enthaltende Fungizide

(57) Die vorliegende Erfindung betrifft neue cyclische Amine, Verfahren zu deren Herstellung, ihre Verwendung als Fungizide, fungizide Mittel, die die neuen Wirkstoffe enthalten,Verfahren zur Herstellung solcher Fungizide sowie Verfahren zur Bekämpfung von Schadpilzen mit diesen Fungiziden.

Es ist bekannt, N-Tridecyl-2,6-dimethylmorpholin als Fungizid zu verwenden (DE 1 164 152).

Es wurde nun gefunden, daß Verbindungen der Formel I

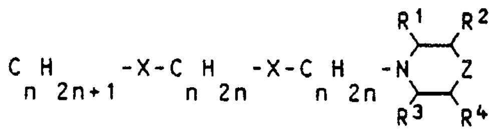

I

worin

die $C_nH_{2n+1}$- und die $C_nH_{2n}$-Einheiten geradkettig oder verzweigt sind und n die Zahlen 2 bis 10, (2,3,4,5,6,7,8,9,10), wobei die einzelnen Werte von n gleich oder verschieden sind,

X Sauerstoff oder Schwefel

Z Sauerstoff oder CH-$R^5$ bedeutet und

$R^1$, $R^2$, $R^3$, $R^4$ und $R^5$ gleich oder verschieden sind und unabhängig voneinander Wasserstoff oder $C_1$-$C_2$-Alkyl (Methyl, Ethyl) bedeuten sowie deren Salze ausgezeichnet wirksam gegen Schadpilze sind und sehr gute Pflanzenverträglichkeit zeigen.

Die neuen Amine der Formel I enthalten ggf. chirale Zentren. Sie werden im allgemeinen als Racemate und gegebenenfalls als Diastereomerengemische erhalten. Einheitliche Diastereomere lassen sich bei einigen der neuen Verbindungen beispielsweise durch Säulenchromatographie oder aufgrund von Löslichkeitsunterschieden in reiner Form isolieren. Aus solchen gereinigten Diastereomeren kann man mit bekannten Methoden einheitliche Racemate und Enantiomere erhalten. Alle diese Verbindungen und Gemische werden von der vorliegenden Erfindung umfaßt. Für die Anwendung der neuen Amine als Fungizide sind sowohl die einheitlichen Diastereomeren bzw. Enantiomeren wie auch deren bei der Synthese anfallenden Gemische geeignet, Bevorzugt werden die letzteren verwendet.

Die Amine der Formel I lassen sich herstellen, indem man

a) eine Verbindung der Formel II mit einem Amin der Formel III umsetzt

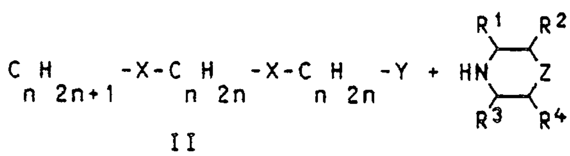

II III

in welcher n, X, Z und $R^{1-5}$ die obengenannten Bedeutungen haben und Y für eine nucleophil verdrängbare Abgangsgruppe, beispielsweise Halogen (Cl, Br) oder Alkyl- oder Arylsulfonyl steht, oder

b) ein Alkylierungsmittel der Formel IV mit einem Amin der Formel V umsetzt

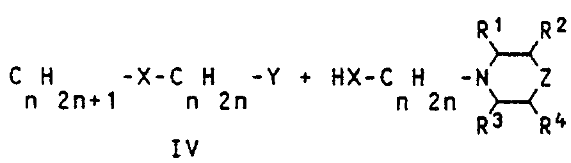

IV V

in welcher X, Z, n und $R^{1-5}$ die obengenannten Bedeutungen haben, oder

c) einen Alkohol oder ein Thiol der Formel IV mit einem Amin der Formel VII umsetzt

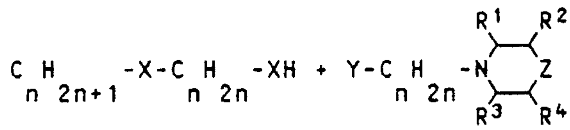 VII

VI

in welcher X, Y, Z, n und $R^{1-5}$ die oben angegebenen Bedeutungen haben, gegebenenfalls in Gegenwart eines Lösungs- oder Verdünnungsmittels und/oder einer anorganischen oder organischen Base und/oder eines Reaktionsbeschleunigers, und die so erhaltenen Verbindungen gegebenenfalls in ihre Salze überführt.

Für alle drei Verfahrensvarianten a), b) und c) kommen als Lösungs- oder Verdünnungsmittel beispielsweise Halogenkohlenwasserstoffe, insbesondere Chlorkohlenwasserstoffe, z.B. Tetrachlorethylen-1,1,2,2- oder 1,1,1,2-Tetrachlorethan, Dichlorpropan, Methylenchlorid, Dichlorbutan, Chloroform, Chlornaphthalin, Tetrachlorkohlenstoff, 1,1,1- oder 1,1,2-Trichlorethan, Trichlorethylen, Pentachlorethan, o-, m-, p-Difluorbenzol, 1,2-Dichlorethan, 1,1-Dichlorethan, 1,2-cis-Dichlorethylen, o-, p-, m-Dibrombenzol, o-, m-, p-Chlortoluol, 1,2,4-Trichlorbenzol; Ether, z.B. Ethylpropylether, Methyl-tert.-butylether, n-Butylethylether, Di-n-butylether, Diisobutylether, Diisoamylether, Diisopropylether, Anisol, Phenetol, Cyclohexylmethylether, Diethylether, Etylenglykoldimethylether, Tetrahydrofuran, Dioxan, Thioanisol, ß,ß,ß'-Dichlordiethylether; Nitrokohlenwasserstoffe wie Nitromethan, Nitroethan, Nitrobenzol, o-, m-, p-Chlornitrobenzol, o-Nitrotoluol; Nitrile wie Acetonitril, Butyronitril, Isobutyronitril, Benzonitril, m-Chlorbenzonitril; aliphatische oder cycloaliphatische Kohlenwasserstoffe, z.B. Heptan, Pinan, Nonan, o-, m-, p-Cymol, Benzinfraktionen innerhalb eines Siedepunktintervalles von 70 bis 190° C, Cyclohexan, Methylcyclohexan, Dekalin, Petrolether, Hexan, Ligroin, 2,2,4-Trimethylpentan, 2,2,3-Trimethylpentan, 2,3,3-Trimethylpentan, Octan; Ester z.B. Ethylacetat, Acetessigester, Isobutylacetat; Amide, z.B. Formamid, Methylformamid, Dimethylformamid; Ketone, z.B. Aceton, Methylethylketon, gegebenenfalls auch Wasser und entsprechende Gemische in Betracht. Als Lösungsmittel können auch Verbindungen der Formel III, IV und V im Überschuß verwendet werden. Zweckmäßig verwendet man das Lösungsmittel in einer Menge von 100 bis 2 000 Gew.%, vorzugsweise von 200 bis 700 Gew.%, bezogen auf Ausgangsstoff II.

Als anorganische oder organische Basen (Säureakzeptoren) für die Umsetzung zu Verbindungen der Formel I können alle üblichen Säurebindemittel verwendet werden. Hierzu gehören vorzugsweise tertiäre Amine, Erdalkaliverbindungen sowie entsprechende Gemische. Es können aber auch Zinkverbindungen verwendet werden. Es kommen z.B. als basische Verbindungen in Frage:
Kaliumhydroxid, Natriumhydroxid, Kaliumcarbonat, Natriumcarbonat, Lithiumhydroxid, Lithiumcarbonat, Natriumbicarbonat, Kaliumbicarbonat, Calciumhydroxid, Calciumoxid, Bariumoxid, Magnesiumhydroxid, Magnesiumoxid, Bariumhydroxid, Calciumcarbonat; Magnesiumcarbonat, Magnesiumbicarbonat, Magnesiumacetat, Zinkhydroxid, Zinkoxid, Zinkcarbonat, Zinkbicarbonat, Zinkacetat, Natriumformiat, Natriumacetat, Trimethylamin, Triethylamin, Tripropylamin, Triisopropylamin, Tributylamin, Triisobutylamin, Tri-sec.-butylamin, Tri-tert.-butylamin, Tribenzylamin, Tri-cyclohexylamin, Triamylamin, Trihexylamin, N,N-Dimethylanilin, N,N-Diethylanilin, N,N-Dipropylanilin, N,N-Dimethyltoluidin, N,N-Dimethyl-p-aminopyridin, N,N-Diethyl-p-aminopyridin, N-Methylpyrrolidin, N-Ethylpyrrolidon, N-Methylpiperidin, N-Ethylpiperidin, N-Methyl-pyrrolidin, N-Ethylpyrrolidin, N-Methylimidazol, N-Ethylimidazol, N-Methylmorpholin, N-Ethylmorpholin, N-Methylhexametylenimin, N-Ethylhexamethylenimin, Pyridin, Chinolin, alpha-Picolin, gamma-Picolin, Isochinolin, Pyrimidin, Acridin, N,N,N'-N'-Tetramethylethylendiamin, N,N,N',N'-Tetraethylethylendiamin, Chinoxalin, Chinazolin, N-Propyldiisopropylamin, N,N-Dimethylcyclohexylamin, 2,6-Lutidin, 2,4-Lutidin, Trifurfurylamin, Triethylendiamin.

Zweckmäßig verwendet man den Säureakzeptor in einem Überschuß oder Unterschuß von bis zu 20 %, bezogen auf den Ausgangsstoff II, IV oder IV.

Als Reaktionsbeschleuniger kommen vorzugsweise Metallhalogenide wie Natriumiodid oder Kaliumiodid in Frage.

Zur Salzbildung mit Verbindungen der Formel I sind alle organischen und anorganischen Säuren geeignet (Säureadditionssalze), soweit sie pflanzenphysiologisch verträgliche Salze bilden. So sind z.B. Chloride, Bromide, Iodide, Sulfate, Phosphate, Acetate, Oxalate, Fumarate, Malonate, Alkylsulfonate, Arylsulfonate und Dodecylbenzolsulfonate zu nennen.

Die Salze werden erhalten, indem man die entsprechenden Säure mit freiem Amin der Formel I, gegebenenfalls in einem inerten Lösungsmittel zusammengibt, das Lösungsmittel abtrennt und den Rückstand gegebenenfalls umkristallisiert.

Die Ausgangsstoffe der Formel II, in welcher Y für Chlor oder Brom steht, sind neu. Sie lassen sich

nach an sich bekannten Methoden darstellen.

Sie können beispielsweise durch Umsetzung (d) von Alkoholen oder Thiolen der Formel IV mit 1, Omega-Dihalogenderivaten der Formel VIII

$Y-C_nH_{2n}-Y$ VIII,

in welcher n und Y die oben angegebenen Bedeutungen haben, gegebenenfalls in Gegenwart eines oder mehreren Lösungs- und Verdünnungsmitteln und/oder anorganischen Basen und/oder eines Phasentransfer-Katalysators hergestellt werden.

Die Umsetzung (d) wird zweckmäßig in gegenüber der Reaktionsteilnehmern inerten Lösungsmitteln, z.B. Toluol, 1,2-Dimethoxyethan, Tetrahydrofuran, Dioxan, Methylenchlorid, Dimethylformaid, Wasser oder deren Gemischen durchgeführt. Als Lösungsmittel können auch die Verbindungen der Formel VIII im Überschuß verwendet werden.

Als säurebindende Mittel kommen z.B. anorganische Basen wie Hydride, Hydroxide, Carbonate, Borate oder Phosphate von Alkali- und Erdalkalimetallen, beispielsweise Natriumhydrid, Natrium- und Kaliumhydroxid, Calciumoxid, Natrium- und Kalium-carbonat und -hydrogencarbonat, Magnesium-, Calcium- oder Bariumcarbonat oder Natrium- und Kaliumphosphate in Frage.atriumhydrid, Natrium- und Kaliumhydroxid, Calciumoxid, Natrium- und Kalium-carbonat und -hydrogencarbonat, Magnesium-, Calcium- oder Bariumcarbonat oder Natrium- und Kaliumphosphate in Frage. Als Phasentransfer-Katalysatoren kommen vorzugsweise quaternäre Ammonium- und Phosphoniumsalze wie Tetrabutylammonium-chlorid, -hydrogensulfat, -hydroxid, -bromid oder -iodid, Benzyltriethylammoniumchlorid, Cetyltrimethylammoniumchlorid, Benzyltriphenylphosphoniumchlorid oder Kronenether wie 12-Krone-4, 15-Krone-5, 18-Krone-6, Dibenzo-18-krone-6 oder Dicyclohexano-18-krone-6 in Frage.

Die Umsetzungen a), b), c) und d) werden im allgemeinen bei Temperaturen zwischen 0 und 100 °C, drucklos oder unter Druck, kontinuierlich oder diskontinuierlich durchgeführt.

Zwischenprodukte wie z.B. die Alkylierungsreagenzien IV, die Amine III, V und VII und die Alkohole bzw. Thiole VI sind bekannt.

Vorschrift 1

Herstellung des Zwischenproduktes

Unter kräftigem Rühren wird eine Mischung aus 98,5 g (0,675 Mol) 1-(Isobutoxy)-2-butan-2-ol, 350 ml 1,4-Dichlorbutan, 10 g Tetrabutylammoniumhydrogensulfat und 250 g 50 %iger (Gew.-%), wäßriger Natriumhydroxidlösung 24 Std. auf 50 °C erwärmt. Die Mischung wird sodann mit 1 l Wasser versetzt und viermal mit je 300 ml Methylenchlorid extrahiert. Die vereinigten Extrakte werden fünfmal mit je 200 ml Wasser ausgeschüttelt, über Magnesiumsulfat getrocknet und im Vakuum fraktioniert destilliert.

Man erhält 81,2 g (51 % d. Th.) 1-(Isobutoxy)-2-(4′-chlorbutoxy)-butan als farblose Flüssigkeit von Sdp. 142 - 143 °C bei 20 mbar und $n_D^{22}$ = 1,4361.

Beispiel 1

cis-2,6-Dimethyl-4-{4′-1″-[Isobutoxy)-butoxy-2″]-butyl-1′-morpholin

Unter Rühren wird eine Mischung aus 20 g (0,08 Mol) 1-(Isobutoxy)-2-(4′-chlorbutoxy-1′)-butan, 70 ml cis-2,6-Dimethylmorpholin und 2 g Kaliumiodid 24 Stunden auf 90 °C erwärmt. Nach Abkühlen auf 10 °C wird das Reaktionsgemisch nacheinander mit 100 ml Ether und 50 ml 50 %iger wäßriger Natriumhydroxidlösung versetzt. Die organische Phase wird über $Na_2SO_4$ getrocknet und im Vakuum fraktioniert destilliert.

Man erhält 20 g (80 % d. Th.) der Titelverbindung als farbloses Öl vom Sdp. 122 - 123 °C/0,1 mbar und $n_D^{25}$ = 1,4462.

Auf entsprechende Weise wie in Vorschrift 1 lassen sich die folgenden Zwischenprodukte der Formel II herstellen:

Tabelle 1: Zwischenprodukte der Formel $C_nH_{2n+1}-X-C_nH_{2n}-X-C_nH_{2n}-Y$

| $C_nH_{2n+1}$ | X | $C_nH_{2n}$ | X | $C_nH_{2n}$ | Y | Brechungsindex oder Siedepunkt (°C/mbar) |
|---|---|---|---|---|---|---|
| n-Propyl | O | $-(CH_2)_2-$ | O | $-(CH_2)_4-$ | Cl | 78- 90/0,2 |
| n-Propyl | S | $-(CH_2)_2-$ | O | $-(CH_2)_6-$ | Cl | 91- 93/0,15 |
| Isopropyl | O | $-(CH_2)_2-$ | O | $-(CH_2)_4-$ | Cl | 73- 75/0,2 |
| Isopropyl | S | $-(CH_2)_2-$ | O | $-(CH_2)_6-$ | Cl | $n_D^{22}$ 1,4729 |
| Isopropyl | O | $-CH_2-CH(CH_3)-$ | O | $-(CH_2)_4-$ | Cl | 80- 84/0,3 |
| Isopropyl | O | $-CH_2-CH(CH_3)-$ | O | $-(CH_2)_6-$ | Cl | 95- 97/0,3 |
| Isopropyl | O | $-CH_2-CH(C_2H_5)-$ | O | $-(CH_2)_4-$ | Cl | 93- 96/0,3 |
| n-Butyl | O | $-CH_2-CH(CH_3)-$ | O | $-(CH_2)_4-$ | Cl | 88- 91/0,2 |
| n-Butyl | O | $-CH(CH_3)-CH_2-$ | O | $-(CH_2)_4-$ | Cl | 92- 96/0,3 |
| n-Butyl | S | $-CH_2-CH(CH_3)-$ | O | $-(CH_2)_4-$ | Cl | 91- 94/0,1 |
| Isobutyl | O | $-(CH_2)_2-$ | O | $-(CH_2)_6-$ | Cl | $n_D^{22}$ 1,4410 |
| Isobutyl | S | $-(CH_2)_2-$ | O | $-(CH_2)_6-$ | Cl | 89- 92/0,2 |
| Isobutyl | O | $-CH_2-CH(CH_3)-$ | O | $-(CH_2)_4-$ | Cl | 136-139/20 |
| Isobutyl | O | $-CH_2-CH(CH_3)-$ | O | $-(CH_2)_6-$ | Cl | $n_D^{22}$ 1,4382 |
| Isobutyl | O | $-CH_2-CH(C_2H_5)-$ | O | $-(CH_2)_3-$ | Cl | 139-142/20 |
| Isobutyl | O | $-CH_2-CH(C_2H_5)-$ | O | $-(CH_2)_4-$ | Cl | $n_D^{23}$ 1,4361 |
| Isobutyl | S | $-CH_2-CH(C_2H_5)-$ | O | $-(CH_2)_4-$ | Cl | 145-147/20 |
| Isobutyl | O | $-CH_2-CH(C_3H_7)-$ | O | $-(CH_2)_4-$ | Cl | $n_D^{22}$ 1,4381 |
| Isobutyl | O | $-CH_2-CH(C_3H_7)-$ | O | $-(CH_2)_5-$ | Cl | $n_D^{22}$ 1,4381 |
| Isobutyl | O | $-CH_2-CH(C_3H_7)-$ | O | $-(CH_2)_6-$ | Cl | 148-150/20 |
| Isobutyl | O | $-CH_2-CH(C_6H_{13})-$ | O | $-(CH_2)_4-$ | Cl | $n_D^{23}$ 1,4427 |

Tabelle 1 (Fortsetzung)

| $C_nH_{2n+1}$ | X | $C_nH_{2n}$ | X | $C_nH_{2n}$ | Y | Brechungsindex oder Siedepunkt (°C/mbar) |
|---|---|---|---|---|---|---|
| Isobutyl | O | $-CH_2-CH(CH_3)-$ | O | $-(CH_2)_8-$ | Cl | 151-153/17 |
| Isobutyl | O | $-CH_2-CH(CH_3)-$ | O | $-(CH_2)_8-$ | Cl | 150-154/17 |
| n-Hexyl | O | $-CH_2-CH(CH_3)-$ | O | $-(CH_2)_4-$ | Cl | 110-113/0,2 |
| n-Hexyl | O | $-CH_2-CH(C_2H_5)-$ | O | $-(CH_2)_4-$ | Cl | 112-115/0,2 |
| 2,2-Dimethylpropyl-1- | O | $-(CH_2)_2-$ | O | $-(CH_2)_4-$ | Cl | 109-111/0,2 |
| 2,2-Dimethylpropyl-1- | O | $-CH_2(CH_3)-CH_2-$ | O | $-(CH_2)_6-$ | Cl | 120-122/0,3 |
| 3,3-Dimethylbutyl-1- | O | $-(CH_2)_4-$ | O | $-(CH_2)_6-$ | Cl | 123-126/0,2 |
| 3,3-Dimethylbutyl-1- | O | $-CH_2-CH(CH_3)-$ | O | $-(CH_2)_4-$ | Cl | 110-112/0,2 |
| 3,3-Dimethylbutyl-1- | O | $-CH_2-CH(C_2H_5)-$ | O | $-(CH_2)_4-$ | Cl | 113-116/0,2 |
| 2,4-Dimethylpentyl-1- | O | $-(CH_2)_2-$ | O | $-(CH_2)_4-$ | Cl | 150-152/20 |
| 2,4-Dimethylpentyl-1- | O | $-CH_2CH(C_2H_5)-$ | O | $-(CH_2)_4$ | Cl | 112-115/0,2 |
| 3-Heptyl | O | $-CH(CH_3)CH_2$ | O | $-(CH_2)_4-$ | Cl | $n_D^{23}$ 1,4410 |
| 2-Ethyl-4-methyl pentyl-1 | O | $-CH_2-CH(C_2H_5-$ | O | $-(CH_2)_4-$ | Cl | 110-113/0,2 |
| 2-Ethyl-4-methyl pentyl-1 | O | $-CH_2-CH(C_2H_5)-$ | O | $-(CH_2)_6-$ | Cl | 119-123/0,2 |
| 2-Ethylhexyl-1 | O | $-CH_2-CH(C_2H_5)-$ | O | $-(CH_2)_6-$ | Cl | 158-160/17 |
| 2-Ethylhexyl-1 | O | $-CH_2-CH(CH_3)-$ | O | $-(CH_2)_4-$ | Cl | 147-149/17 |
| n-Octyl | O | $-CH_2-CH(C_2H_5)-$ | O | $-(CH_2)_4-$ | Cl | 149-153/20 |
| n-Octyl | O | $-CH_2-CH(C_2H_5)-$ | O | $-(CH_2)_6-$ | Cl | 121-124/0,2 |
| n-Decyl | O | $-CH_2-CH(CH_3)-$ | O | $-(CH_2)_4-$ | Cl | 125-126/0,1 |
| n-Decyl | S | $-CH_2-CH(CH_3)-$ | O | $-(CH_2)_4-$ | Cl | 131-135/0,1 |
| n-Decyl | O | $-CH_2-CH(C_2H_5)-$ | O | $-(CH_2)_4-$ | Cl | 130-134/0,2 |

Tabelle 2 Verbindungen der allgemeinen Formel I

Entsprechend wie in Beispiel 1 können durch Wahl der Ausgangsstoffe und entsprechende Anpassung der Verfahrensbedingungen die nachstehend in Tabelle 2 aufgelisteten Verbindungen erhalten werden.

| Beisp. Nr. | $C_nH_{2n+1}-X-C_nH_{2n}-X-C_nH_{2n}$ | $R^1$ | $R^2$ | $R^3$ | $R^4$ | Z | Brechungsindex oder Sdp. (°C/mbar) |
|---|---|---|---|---|---|---|---|
| 2 | $CH_3(CH_2)_2-O-(CH_2)_4-$ | H | $CH_3$ | H | $CH_3$ | 0 | 133-136/0,3 |
| 3 | $(CH_3)_2CH-O-(CH_2)_2-O-(CH_2)_4-$ | H | $CH_3$ | H | $CH_3$ | 0 | 128-131/0,2 |
| 4 | $(CH_3)_2CH-O-(CH_2)_2-O-(CH_2)_4-$ | H | $CH_3$ | H | $CH_3$ | 0 | 129-132/0,2 |
| 5 | $(CH_3)_2CH-O-(CH_2)_2-O-(CH_2)_4-$ | H | $CH_3$ | H | $CH_3$ | 0 | 125-128/0,1 |
| 6 | $(CH_3)_2CH-O-(CH_2)_2-O-(CH_2)_6-$ | H | $CH_3$ | H | $CH_3$ | 0 | 132-135/0,2 |
| 7 | $(CH_3)_2CH-O-(CH_2)_2-O-(CH_2)_6-$ | H | $CH_3$ | H | $CH_3$ | 0 | 133-136/0,2 |
| 8 | $(CH_3)_2CH-O-(CH_2)_2-O-(CH_2)_4-$ | H | $CH_3$ | H | $CH_3$ | 0 | 142-144/0,3 |
| 9 | $(CH_3)_2CH-O-(CH_2)_2-O-(CH_2)_6-$ | H | $CH_3$ | H | $CH_3$ | 0 | $n^{22}$ 1,4742 |
| 10 | $(CH_3)_2CH-CH_2-O-(CH_2)_2-O-(CH_2)_4-$ | H | $CH_3$ | H | $CH_3$ | 0 | 120-124/0,2 |
| 11 | $(CH_3)_2CH-CH_2-O-CH_2-CH(CH_3)-O-(CH_2)_4-$ | H | $CH_3$ | H | $CH_3$ | 0 | 121-125/0,1 |
| 12 | $(CH_3)_2CH-CH_2-O-CH_2-CH(CH_3)-O-(CH_2)_4-$ | H | $CH_3$ | H | $CH_3$ | 0 | 122-126/0,2 |
| 13 | $(CH_3)_2CH-CH_2-O-CH_2-CH(CH_3)-O-(CH_2)_4-$ | H | $CH_3$ | H | $CH_3$ | 0 | 128-130/0,3 |
| 14 | $(CH_3)_2CH-CH_2-O-CH_2-CH(CH_3)-O-(CH_2)_4-$ | H | $CH_3$ | H | $CH_3$ | 0 | 125-128/0,2 |
| 15 | $(CH_3)_2CH-CH_2-O-CH_2-CH(CH_3)-O-(CH_2)_4-$ | H | $CH_3$ | H | $CH_3$ | 0 | 129-133/0,3 |
| 16 | $(CH_3)_2CH-CH_2-O-(CH_2)_2-O-(CH_2)_6-$ | H | $CH_3$ | H | $CH_3$ | 0 | $n^{25}$ 1,4501 |
| 17 | $(CH_3)_2CH-CH_2-O-(CH_2)_2-O-(CH_2)_6-$ | H | $CH_3$ | H | $CH_3$ | 0 | 142-145/0,1 |
| 18 | $(CH_3)_2CH-CH_2-O-CH_2-CH(CH_3)-O-(CH_2)_6-$ | H | $CH_3$ | H | $CH_3$ | 0 | $n^{22}$ 1,4493 |
| 19 | $(CH_3)_2CH-CH_2-O-CH_2-CH(CH_3)-O-(CH_2)_6-$ | H | $CH_3$ | H | $CH_3$ | 0 | 122-125/0,1 |
| 20 | $(CH_3)_2CH-CH_2-O-CH_2-CH(CH_3)-O-(CH_2)_6-$ | H | $CH_3$ | H | $CH_3$ | 0 | 128-131/0,2 |
| 21 | $(CH_3)_2CH-CH_2-O-CH_2-CH(CH_3)-O-(CH_2)_6-$ | H | $CH_3$ | H | $CH_3$ | 0 | 123-126/0,1 |

Tabelle 2 (Fortsetzung)

| Beisp. Nr. | $C_nH_{2n+1}-X-C_nH_{2n}-X-C_nH_{2n}$ | $R^1$ | $R^2$ | $R^3$ | $R^4$ | Z | Brechungsindex oder Sdp. (°C/mbar) |
|---|---|---|---|---|---|---|---|
| 22 | $(CH_3)_2CH-CH_2-O-CH_2-CH(CH_3)-O-(CH_2)_6-$ | H | $CH_3$ | H | $CH_3$ | 0 | 135-137/0,2 |
| 23 | $(CH_3)_2CH-CH_2-O-CH_2-CH(C_3H_7)-O-(CH_2)_4-$ | H | $CH_3$ | H | $CH_3$ | 0 | $n^{23}$ 1,4486 |
| 24 | $(CH_3)_2CH-CH_2-O-CH_2-CH(C_3H_7)-O-(CH_2)_6-$ | H | $CH_3$ | H | $CH_3$ | 0 | $n^{23}$ 1,4509 |
| 25 | $(CH_3)_2CH-CH_2-O-CH_2-CH(C_6H_{13})-O-(CH_2)_4-$ | H | $CH_3$ | H | $CH_3$ | 0 | 157-160/0,3 |
| 26 | $(CH_3)_2CH-CH_2-O-CH_2-CH(CH_3)-O-(CH_2)_8-$ | H | $CH_3$ | H | $CH_3$ | 0 | 156-159/0,2 |
| 27 | $CH_3-(CH_2)_5-O-CH_2-CH(CH_3)-O-(CH_2)_4-$ | H | $CH_3$ | H | $CH_3$ | 0 | 146-148/0,2 |
| 28 | $CH_3-(CH_2)_5-O-CH_2-CH(CH_3)-O-(CH_2)_4-$ | H | $CH_3$ | H | $CH_3$ | 0 | 140-142/0,2 |
| 29 | $CH_3-(CH_2)_5-O-CH_2-CH(C_2H_5)-O-(CH_2)_4$ | H | $CH_3$ | H | $CH_3$ | 0 | 140-143/0,1 |
| 30 | $CH_3-(CH_2)_5-O-CH_2-CH(C_2H_5)-O-(CH_2)_4$ | H | $CH_3$ | H | $CH_3$ | 0 | 148-151/0,2 |
| 31 | $(CH_3)_3C-CH_2-O-(CH_2)_2-O-(CH_2)_4-$ | H | $CH_3$ | H | $CH_3$ | 0 | 122-125/0,1 |
| 32 | $(CH_3)_3C-CH_2-O-CH_2(CH_3)-CH_2-O-(CH_2)_6-$ | H | $CH_3$ | H | $CH_3$ | 0 | 147-149/0,3 |
| 33 | $(CH_3)_3C-CH_2-O-(CH_2)_4-O-(CH_2)_6-$ | H | $CH_3$ | H | $CH_3$ | 0 | 157-160/0,2 |
| 34 | $(CH_3)_3C-CH_2-O-CH_2CH(CH_3)-O-(CH_2)_4-$ | H | $CH_3$ | H | $CH_3$ | 0 | 125-128/0,1 |
| 35 | $(CH_3)_3C-CH_2-CH_2-O-CH_2CH(C_2H_5)-O-(CH_2)_4-$ | H | $CH_3$ | H | $CH_3$ | 0 | 127-130/0,1 |
| 36 | $(CH_3)_2CH-CH_2-CH(CH_3)-CH_2-O-(CH_2)_2-O-(CH_2)_4$ | H | $CH_3$ | H | $CH_3$ | 0 | 147-150/0,2 |
| 37 | $(CH_3)_2CH-CH_2-CH(CH_3)-CH_2-O-CH_2-CH(C_2H_5)-O-(CH_2)_4-$ | H | $CH_3$ | H | $CH_3$ | 0 | 155-158/0,3 |
| 38 | $CH_3-(CH_2)_2-CH(C_3H_7)-O-CH(CH_3)CH_2-O-(CH_2)_4-$ | H | $CH_3$ | H | $CH_3$ | 0 | $n^{22}$ 1,4502 |
| 39 | $(CH_3)_2-CH_2-CH(C_2H_5)-CH_2-O-CH_2CH(C_2H_5)-O-(CH_2)_4-$ | H | $CH_3$ | H | $CH_3$ | 0 | 161-163/0,3 |
| 40 | $(CH_3)_2-CH_2-CH(C_2H_5)-CH_2-O-CH_2CH(C_2H_5)-O-(CH_2)_4-$ | H | $CH_3$ | H | $CH_3$ | 0 | 147-149/0,3 |
| 41 | $CH_3-(CH_2)_3-CH(C_2H_5)-CH_2-O-CH_2CH(CH_3)-O-(CH_2)_4-$ | H | $CH_3$ | H | $CH_3$ | 0 | 153-155/0,2 |
| 42 | $CH_3-(CH_2)_3-CH(C_2H_5)-CH_2-O-CH_2CH(CH_3)-O-(CH_2)_4-$ | H | $CH_3$ | H | $CH_3$ | 0 | 140-142/0,2 |
| 43 | $CH_3-(CH_2)_3-CH(C_2H_5)-CH_2-O-CH_2-CH(C_2H_5)-O-(CH_2)_4-$ | H | $CH_3$ | H | $CH_3$ | 0 | 145-147/0,2 |
| 44 | $CH_3-(CH_2)_3-CH(C_2H_5)-CH_2-O-CH_2-CH(C_2H_5)-O-(CH_2)_4-$ | H | $CH_3$ | H | $CH_3$ | 0 | 155-158/0,2 |

Tabelle 2 (Fortsetzung)

| Beisp. Nr. | $C_nH_{2n+1}-X-C_nH_{2n}-X-C_nH_{2n}$ | $R^1$ | $R^2$ | $R^3$ | $R^4$ | Z | Brechungsindex oder Sdp. (°C/mbar) |
|---|---|---|---|---|---|---|---|
| 45 | $CH_3-(CH_2)_7-O-CH_2-CH(C_2H_5)-O-(CH_2)_2-O-(CH_2)_4-$ | H | $CH_3$ | H | $CH_3$ | O | 156-158/0,2 |
| 46 | $CH_3-(CH_2)_7-O-CH_2-CH(C_2H_5)-O-(CH_2)_6-$ | H | $CH_3$ | H | $CH_3$ | O | 149-153/0,1 |
| 47 | $CH_3-(CH_2)_7-O-CH_2-CH(C_2H_5)-O-(CH_2)_6-$ | H | $CH_3$ | H | $CH_3$ | O | 140-142/0,1 |
| 48 | $CH_3-(CH_2)_9-O-CH_2-CH(CH_3)-O-(CH_2)_4-$ | H | $CH_3$ | H | $CH_3$ | O | 147-149/0,2 |
| 49 | $CH_3-(CH_2)_9-S-CH_2-CH(C_2H_5)-O-(CH_2)_4-$ | H | $CH_3$ | H | $CH_3$ | O | 165-169/0,1 |
| 50 | $CH_3-(CH_2)_9-O-CH_2-CH(C_2H_5)-O-(CH_2)_4-$ | H | $CH_3$ | H | $CH_3$ | O | 150-156/0,1 |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten und Deuteromyceten, aber auch der Phycomyceten aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,

Erysiphe cichoracearum bzw. Sphaerotheca fuligenea an Kürbisgewächsen,
Podosphaera leucotricha an Äpfeln,
Uncinula necator an Reben,
Puccinia-Arten an Getreide,
Venturia inaequalis (Schorf) an Äpfeln,
Septoria nodorum an Weizen,
Botrytis cinerea (Grauschimmel) an Erdbeeren, Reben,
Pseudocercosporella herpotrichoides an Weizen, Gerste,
Pyricularia oryzae an Reis,
Alternaria solani an Kartoffeln, Tomaten,
Fusarium- und Verticillum-Arten an verschiedenen Pflanzen,
Plasmopara viticola an Reben,
Pyrenophora teres an Gerste.

Die Verbindungen werden angewendet, indem man die Pflanzen mit dem Wirkstoff besprüht oder bestäubt oder die Samen der Pflanzen mit den Wirkstoffen behandelt. Die Anwendung erfolgt vor und nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z. B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z. B. Xylol, Benzol) chlorierte Aromaten (z. B. Chlorbenzole), Paraffine (z. B. Erdölfraktionen), Alkohole (z. B. Methanol, Butanol), Ketone (z. B. Cyclohexanon), Amine (z. B. Ethanolamin, Dimethylformamid) und Wasser; Trägerstoffe wie natürliche Gesteinsmehle, (z. B. Kaoline, Tonerden, Talkum, Kreide) und synthetische Gesteinsmehle (z. B. hochdisperse Kieselsäure, Silikate); Emulgiermittel wie nichtionogene Emulgatoren (z.B. Polyoxyethylen-Fettalkohol-Ether, Alkylsulfonate und Arylsulfonate) und Dispergiermittel-wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I.

Man vermischt 90 Gewichtsteile der Verbindung Nr. 1 mit 10 Gewichtsteilen N-Methyl-alpha-pyrrolidon und erhält eine Lösung, die zur Anwendung in Form kleinster Tropfen geeignet ist.

II.

20 Gewichtsteile der Verbindung Nr. 23 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

III.

20 Gewichtsteile der Verbindung Nr. 1 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV.

20 Gewichtsteile der Verbindung Nr. 23 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V.

80 Gewichtsteile der Verbindung Nr. 1 werden den mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfitablauge und 7 Gewichtsteilen pulverförmigem Kieselsäuregel gut vermischt und in einer Hammermühle vermahlen. Durch feines Verteilen der Mischung in Wasser erhält man eine Spritzbrühe.

VI.

3 Gewichtsteile der Verbindung Nr. 23 werden mit 97 Gewichtsteilen feinteiligem Kaolin innig vermischt. Man erhält auf diese Weise ein Stäubemittel, das 3 Gew.% des Wirkstoffs enthält.

VII.

30 Gewichtsteile der Verbindung Nr. 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII.

40 Gewichtsteile der Verbindung Nr. 23 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit Wasser erhält man eine wäßrige Dispersion.

XI.

20 Teile der Verbindung Nr. 1 werden mit 2 Teilen Calciumsalz der Dodecylbezolsulfonsäure, 8 Teilen Fettalkoholpolyglykolether, 2 Teilen Natriumsalz eines Phenolsulfonsäure-harnstoff-formaldehyd-Kondensates und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z. B. Herbiziden, Insektiziden, Wachstumsregulatoren und Fungiziden oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken. Fungizide, die mit den neuen Verbindungen kombiniert werden können, sind beispielsweise:

Schwefel
Dithiocarbamate und deren Derivate, wie
Ferridimethyldithiocarbamat
Zinkdimethyldithiocarbamat
Zinkethylenbisdithiocarbamat
Manganethylenbisdithiocarbamat
Mangan-Zink-ethylendiamin-bis-dithiocarbamat
Tetramethylthiuramdisulfide
Ammoniak-Komplex von Zink-(N,N′-ethylen-bis-dithiocarbamat)
Ammoniak-Komplex von Zink-(N,N′-propylen-bis-dithiocarbamat)
Zink-(N,N′-propylen-bis-dithiocarbamat) N,N′-Polypropylen-bis-(thiocarbomyl)-disulfid
Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat
5-Nitroisophthalsäure-di-isopropylester,
heterocyclische Strukturen, wie
2-Heptadecyl-2-imidazolon-acetat
2,4-Dichlor-6-(o-chloranilino)-s-triazin
0,0-Diethyl-phthalimidophosphonothioat
5-Amino-1-(bis-(dimethylamino)-phosphinyl)-3-phenyl-1,2,4-triazol
2,3-Dicyano-1,4-dithiaanthrachinon
2-Thio-1,3-dithiolo[4,5b]chinoxalin
1-(Butylcarbamoyl)-2-benzimidazol-carbaminsäuremethylester
2-Methoxycarbonylamino-benzimidazol

2-(Furyl-(2)-benzimidazol
2-(Thiazolyl-(4)-benzimidazol
N-(1,1,2,2-Tetrachlorethylthio)-tetrahydrophthalimid
N-Trichlormethylthio-tetrahydrophthalimid
N-Trichlormethylthio-phthalimid
N-Dichlorfluormethylthio-N′,N′-dimethyl-N-phenyl-schwefelsäurediamid
5-Ethoxy-3-trichlormethyl-1,2,3-thiadiazol
2-Rhodanmethylthiobenzthiazol
1,4-Dichlor-2,5-dimethoxybenzol
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin
2,3-Dihydro-5-carboxanilido-6-methyl-1,4-oxathiin-4,4-dioxid
2-Methyl-5,6-dihydro-4-H-pyran-3-carbonsäure-anilid
2-Methyl-furan-3-carbonsäureanilid
2,5-Dimethyl-furan-3-carbonsäureanilid
2,4,5-Trimethyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
2-Methyl-benzoesäure-anilid
2-Iod-benzoesäure-anilid
N-Formyl-N-morpholin-2,2,2-trichlorethylacetal
Piperazin-1,4-diylbis-(1-(2,2,2-trichlor-ethyl)-formamid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
N-3-(p-tert.-Butylphenyl)-2-methylpropyl-cis-2,6-dimethylmorpholin
N-3-(p-tert.-Butylphenyl)-2-methylpropyl-piperidin
1-2-(2,4-Dichlorphenyl)-4-ethyl-1,3-dioxolan-2-yl-ethyl-1-H-1,2 ,4-triazol
12-(2,4-Dichlorphenyl)-4-n-propyl-1,3-dioxolan-2-yl-ethyl-1-H-1 ,2,4-triazol
N-(n-Propyl)-N-(2,4,6-trichlorphenoxyethyl)-N′-imidazol-yl-harnstoff
1-(4-Chlorphenoxy)-3,3-dimethyl-1-(1-H-1,2,4-triazol-1-yl)-2-butanon
1-(4-Chlorphenoxy)-3,3-dimethyl-1-(1-H-1,2,4-triazol-1-yl)-2-butanol
alpha-(2-Chlorphenyl)-alpha-(4-chlorphenyl)-5-pyrimidin-methanol
5-Butyl-2-dimethylamino-4-hydroxy-6-methyl-pyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol
sowie verschiedene Fungizide, wie
Dodecylguanidinacetat
3-(3-(3,5-Dimethyl-2-oxycyclohexyl)-2-hydroxyethyl)-gluataramid
Hexachlorbenzol
DL-Methyl-N-(2,6-dimethyl-phenyl)-N-furoyl(2)-alaninat
DL-N-(2,6-Dimethyl-phenyl)-N-(2′-methoxyacetyl)-alaninmethylester
N-(2,6-Dimethylphenyl)-N-chloracetyl-D,L-2-aminobutyrolacton
DL-N-(2,6-Dimethylphenyl)-N-(phenylacetyl)-alaninmethylester
5-Methyl-5-vinyl-3-(3,5-dichlorphenyl)2,4-dioxo-1,3-oxazolidin
3-(3,5-Dichlorphenyl)-5-methyl-5-methoxymethyl-1,3-oxazolidin-2,4-dion
3-(3,5-Dichlorphenyl)-1-isopropylcarbamoylhydantoin
N-(3,5-Dichlorphenyl)-1,2-dimethylcyclopropan-1,2-dicarbonsäureimid
2-Cyano-N-(ethylaminocarbonyl)-2-methoximino-acetamid
1-(2-(2,4-Dichlorphenyl)-pentyl)-1-H-1,2,4-triazol
2,4-Difluor-alpha-(1-H-1,2,4-triazolyl-1-methyl)-benzhydrylalkohol

Vergleichsbeispiel

Für das folgende Vergleichsbeispiel wurde als bekannter Vergleichswirkstoff die Verbindung (A) N-Tridecyl-2,6-dimethylmorpholin - bekannt aus DE 1 164 152 - verwendet.

Wirksamkeit gegen Pseudocercosporella herpotrichoides

Weizenpflanzen der Sorte "Frühgold" wurden im Ein-Blatt-Stadium mit wäßrigen Wirkstoffaufbereitungen, die 80 % Wirkstoff und 20 % Emulgiermittel in der Trockensubstanz enthielten, bis zur Tropfnässe besprüht. 24 Stunden später wurden diese Pflanzen mit einer Sporensuspension von Pseudocercosporella herpotrichoides inokuliert. Zur optimalen Entwicklung der Pflanzenkrankheiten wurden die Pflanzen anschließend für 1 Woche in eine Klimakammer mit 16 - 18° C und einer rel. Luftfeuchtigkeit von mehr als 95 % gestellt. Dann wurden die Pflanzen für zwei weitere Wochen im Gewächshaus bei 15 - 17° C kultiviert. Die Auswertung erfolgte durch Beurteilung der Pilzentwicklung am unteren Teil des Pflanzenstengels.

Das Ergebnis zeigt, daß die Wirkstoffe 1 und 23 bei der Anwendung als 0,1 %ige (Gew.%) Spritzbrühe eine bessere fungizide Wirkung zeigen (94 %) als der bekannte Vergleichswirkstoff A (38 %).

**Ansprüche**

1. Amine der allgemeinen Formel I

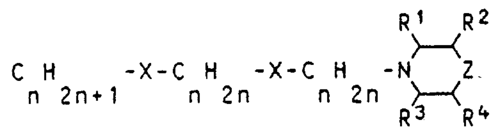

I

worin

n die Zahlen 2 bis 10,

X Sauerstoff oder Schwefel,

Z Sauerstoff oder CH-$R_5$ bedeutet und

$R^1$, $R^2$, $R^3$, $R^4$ und $R^5$ Wasserstoff oder Alkyl bedeuten, sowie deren Salze.

2. Verfahren zur Herstellung von Aminen gemäß Anspruch 1, dadurch gekennzeichnet, daß man

a) eine Verbindung der Formel II mit einem Amin der Formel III umsetzt

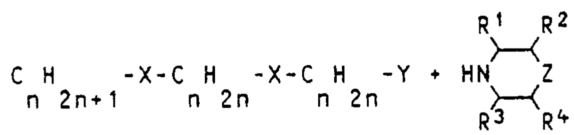

II III

in welcher n, X, Z und $R^{1-5}$ die obengenannten Bedeutungen haben und Y für eine nucleophil verdrängbare Abgangsgruppe steht, oder

b) ein Alkylierungsmittel der Formel IV mit einem Amin der Formel V umsetzt

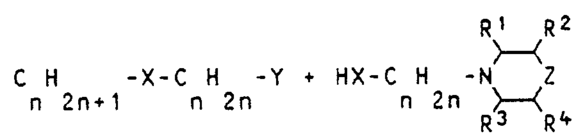

IV V

in welcher X, Y, Z, n und $R^{1-5}$ die obengenannten Bedeutungen haben, oder

c) einen Alkohol oder ein Thiol der Formel VI mit einem Amin der Formel VII umsetzt

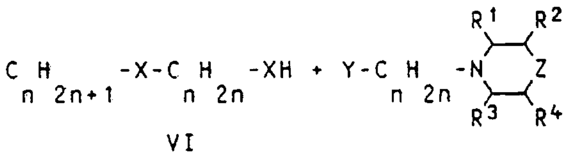

VII

VI

in welcher X, Y, Z, n und $R^{1-5}$ die oben angegebenen Bedeutungen haben, und die erhaltenen Verbindungen gegebenenfalls in ihre Salze überführt.

3. Fungizid, enthaltend einen festen oder flüssigen Trägerstoff und eine fungizid wirksame Menge eines Amins der allgemeinen Formel I

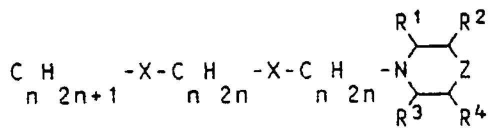

I

worin
n die Zahlen 2 bis 10,
X Sauerstoff oder Schwefel,
Z Sauerstoff oder CH-$R_5$ bedeutet und
R', $R^2$, $R^3$, $R^4$ und $R^5$ Wasserstoff oder Alkyl bedeuten, oder dessen Salzes.

4. Verfahren zur Herstellung eines Fungizids, dadurch gekennzeichnet, daß man einen festen oder flüssigen Trägerstoff vermischt mit einer Verbindung gemäß Anspruch 1.

5. Verfahren zur Bekämpfung von Pilzen, dadurch gekennzeichnet, daß man die Pilze oder die vor Pilzbfall zu schützenden Pflanzen, Boden oder Saatgüter behandelt mit einer fungizid wirksamen Menge eines Amins der allgemeinen Formel I

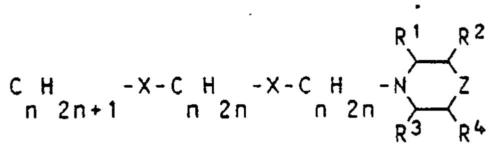

I

worin
n die Zahlen 2 bis 10,
X Sauerstoff oder Schwefel,
Z Sauerstoff oder CH-$R_5$ bedeutet und
R', $R^2$, $R^3$, $R^4$ und $R^5$ Wasserstoff oder Alkyl bedeuten, oder dessen Salzes.

6. Verbindung der Formel I gemäß Anspruch 1, in welcher der Rest $C_nH_{2n+1}$-X-$C_nH_{2n}$-X-$C_nH_{2n}$- die Bedeutung $(CH_3)_2CH$-$CH_2$-O-$(CH_2)_4$-O-$(CH_2)_4$-hat und $R^1$ und $R^3$ Wasserstoff, $R^2$ und $R^4$ Methyl und Z Sauerstoff bedeuten.

7. Verbindung der Formel I gemäß Anspruch 1, in welcher der Rest $C_nH_{2n+1}$-X-$C_nH_{2n}$-X-$C_nH_{2n}$- die Bedeutung $(CH_3)_2CH$-$CH_2$-O-$(CH_2)_5$-$(CH_2)_4$-hat und $R^1$ und $R^2$ Wasserstoff, $R^2$ und $R^4$ Methyl und Z Sauerstoff bedeuten.